# EUROPEAN PATENT APPLICATION

(11) **EP 4 552 633 A1**
(43) Date of publication of application: **14.05.2025**
(21) Application number: 24210689.6
(22) Date of filing: 04.11.2024
(51) Int. Cl.: A61K 9/14, A61K 9/20, A61K 31/5377, A61P 7/04

(54) **PHARMACEUTICAL COMPOSITIONS COMPRISING AMORPHOUS SOLID SOLUTIONS OF RIVAROXABAN AND COPOVIDONE AND PREPARATION METHODS THEREOF**

(30) Priority: 10.11.2023 GB 202317232
(71) Applicant: Zentiva K.S., 102 37 Praha 10 (CZ)
(72) Inventor: OBADALOVÁ, Iva, 198 00 Prague (CZ); KREJCIK, Lukas, 190 17 Prague (CZ); SVOBODOVA, Jaroslava, 250 84 Krenice (CZ); TKADLECOVÁ, Marcela, 163 00 Prague (CZ); BOSAK, Jan, 390 02 Tábor (CZ); CERNA, Igor, 169 00 Prague (CZ); HRINOVA, Erika, 104 00 Prague (CZ)
(74) Representative: Reddie & Grose LLP

(57) **Abstract**

The present invention relates to a pharmaceutical composition comprising an amorphous solid solution of rivaroxaban and copovidone, povidone or crospovidone, wherein the weight ratio of rivaroxaban to copovidone, povidone or crospovidone is 1:4.1 to 1:7; to the use of said pharmaceutical composition in the treatment of a subject suffering from at least one of deep vein thrombosis and pulmonary embolism; and to a hot melt extrusion method for preparing such pharmaceutical composition.

## Description

### Field of the Invention

The present invention relates to a pharmaceutical composition comprising an amorphous solid solution of rivaroxaban and copovidone, povidone or crospovidone, wherein the weight ratio of rivaroxaban to copovidone, povidone or crospovidone is 1:4.1 to 1:7.

The present invention relates to a pharmaceutical composition comprising an amorphous solid solution of rivaroxaban and copovidone, wherein the weight ratio of rivaroxaban to copovidone is 1:4.1 to 1:6.

The present invention relates also to the use of said pharmaceutical composition in the treatment of a subject suffering from at least one of deep vein thrombosis and pulmonary embolism, wherein the subject is in a fasted state.

Furthermore, the present invention relates to hot melt extrusion method for preparing said pharmaceutical composition.

### Background

Rivaroxaban (S)-5-Chloro-N-((2-oxo-3-(4-(3-oxomorpholino)phenyl)oxazolidin-5-yl)methyl)thiophene-2-carboxamide is obtained by condensation of the carboxy group of 5-chlorothiophene-2-carboxylic acid with the amino group of (S)-4-(4-(5-(aminomethyl)-2-oxooxazolidin-3-yl)phenyl)morpholin-3-one.

Rivaroxaban is an anticoagulant agent and the active pharmaceutical ingredient (API) in Bayer's Xarelto^{®} tablets, which is used to treat deep vein thrombosis (DVT) and pulmonary embolism (PE). Rivaroxaban inhibits free factor Xa, an enzyme involved in the production of thrombin, which is central to the process of blood clotting. By blocking factor Xa, rivaroxaban decreases the level of thrombin and reduces the risk of blood clots forming in the veins and arteries. Rivaroxaban also prevents existing blood clots from getting larger.

The approved strength of film-coated tablets of Xarelto^{®} are 2.5 mg, 10 mg, 15 mg and 20 mg. The approved excipients part of the tablets core are microcrystalline cellulose, croscarmellose sodium, lactose monohydrate, hypromellose (2910), sodium laurilsulfate, and magnesium stearate; while the approved excipients part of the film-coat are macrogol (3350), hypromellose (2910), titanium dioxide (E 171), and iron oxide yellow (E 172).

Rivaroxaban is classified as a Biopharmaceutical Classification II (BCS Class II) drug and has poor solubility (7 µg/ ml) in aqueous solution.

The pharmacokinetics of drugs is driven by, among other factors, drug absorption. An orally administered drug has to first dissolve in gastrointestinal fluids before it can be absorbed into the blood stream and reach the site of action. Dissolution is the process by which a solute dissolves into the solvent to form a solution and is regulated by the solubility of the solute in the solvent, which represents the maximum concentration of a solute that can dissolve in a solvent. Absorption is the process of drug movement from the site of drug administration to systemic circulation. Drug absorption is quantified in terms of bioavailability. Bioavailability is the extent to which absorption occurs. In other words, bioavailability is the fraction of the administered drug that reaches systemic circulation. Various factors impede or enhance absorption including solubility, rate of dissolution and permeability of the API. Therefore, in order to show good bioavailability, an orally administered API should have good solubility and/or a fast rate of dissolution in the gastrointestinal tract and plasma as well as good permeability across the biological membrane.

Within the framework of human pharmaceuticals, drugs can be classified into one of the following four BCS classes:
- Class I: high solubility, high permeability - generally very well-absorbed compounds;
- Class II: low solubility, high permeability - exhibits dissolution rate-limited absorption;
- Class III: high solubility, low permeability - exhibits permeability-limited absorption;
- Class IV: low solubility, low permeability - very poor oral bioavailability.

Factors that increase the rate of dissolution and/or solubility of a drug are important for BCS class II drugs, such as rivaroxaban, and class IV drugs. The rate and extent of absorption can be altered by food. Food can increase solubility by increasing the volume of fluids into which a drug can be solubilized, increasing the lipophilicity of said fluids, and changing the pH of said fluids. After a meal, the volume of intestinal fluids increases two- to threefold, which increases the amount of drug that may be solubilized. For this reason, 15 mg and 20 mg Xarelto^{®} tablets need to be administered to subjects in a fed state following a meal.

Due to this food dependency, BCS class II and IV drugs carry the risk of under dosing if a subject does not eat prior to drug administration since absorption of the drug is compromised. Over a prolonged period of time, this can have serious consequences. Accordingly, there is a need to improve the bioavailability of BCS class II and IV drugs, in order to remove their dependency on being administered together with food. There is also a need to develop rivaroxaban formulations with diminished food effect to ensure reliable absorption and thus to improve patients' adherence with the treatment.

Solubility-enhancing amorphous solid dispersions or solutions can aid the absorption of hydrophobic, poorly soluble drugs. In amorphous solid dispersions or solutions the API is dispersed within an excipient matrix in a substantially amorphous form. Many crystalline drugs can be prepared in an amorphous form and can be physically stabilized by the presence of an appropriate polymer. With the drug in an amorphous form, no energy is required to break the drug crystal lattice so, relative to the crystalline form, the amorphous form of many poorly water-soluble drugs can achieve substantially higher solubility and markedly faster dissolution. However, in order to achieve a homogenous single-phase, the drug needs to either be dissolved in the polymer below the equilibrium solubility of the drug or it needs to be miscible with the polymer at given storage conditions. The solid-state physical instabilities associated with amorphous solid solutions include amorphous-amorphous phase separation and/or the conversion of the amorphous drug to a crystalline form, both of which negate the solubility advantage of amorphous solid solutions. Amorphous-amorphous phase separation is a phenomenon wherein distinct drug-rich and polymer-rich amorphous phases are formed throughout the amorphous solid solution matrix, which initially consisted of homogenous mix of drug and polymer. Since amorphous-amorphous phase separation reduces the effect of the polymer, there is the need to find amorphous solutions that balance consistent bioavailability with formulation stability.

### Description of the Figures

Fig. 1: dissolution profile at 900 mL of dissolution media simulated human fasted state of intestinal fluid pH 6,5 (FaSSIF).
Fig. 2: dissolution profile at 900 mL of dissolution media simulated human fed state of intestinal fluid pH 5 (FeSSIF).

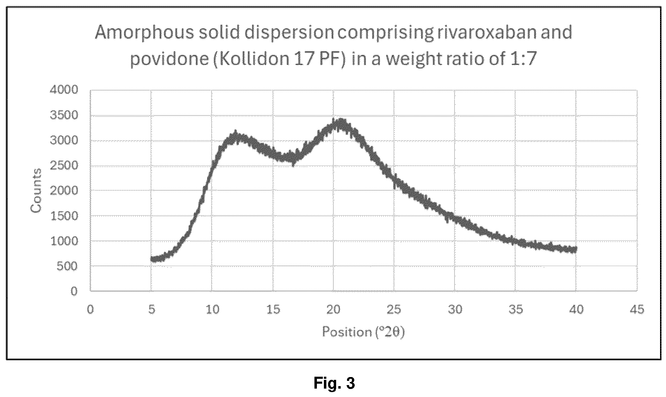

Fig. 3: X-ray powder diffraction (XRD) diffractogram of an amorphous solid dispersion comprising rivaroxaban and povidone (Kollidon^{®} 17 PF) in a weight ratio of 1:7, according to Example 4.

### Detailed Description

The present invention provides a pharmaceutical composition with an optimized weight ratio of API to a pharmaceutically acceptable polymeric excipient showing a high degree of solubility and bioavailability of rivaroxaban.

As used herein, the term "pharmaceutically acceptable polymeric excipient" defines a class of compounds that increase the bioavailability and improve the stability of the API.

The present invention provides a pharmaceutical composition comprising an amorphous solid solution of rivaroxaban and copovidone, povidone or crospovidone, wherein the weight ratio of rivaroxaban to copovidone, povidone or crospovidone is 1:4.1 to 1:7.

As used herein, the term "amorphous solid solution" indicates a monophasic system in an amorphous state, with a no long-range order. In such system the API is molecularly dissolved in the solid excipient matrix, and is distributed at random between the excipient molecules.

As used herein, the term "copovidone" refers to a copolymer of 1-vinyl-2-pyrrolidone and vinyl acetate in a ratio 6:4 by weight. Non limiting examples of copovidone may include polyvinylpyrrolidone-vinyl acetate copolymer; Kollidon^{®} VA 64 and Plasdone^{®} S-630.

As used herein, the term "povidone" refers to poly(1-vinyl-2-pyrrolidone), but may also be referred to as povidonum, polyvidone or PVP. Non-limiting examples of povidone may include Kollidon^{®} 12 PF; Kollidon^{®} 17 PF; Kollidon^{®} 25; Kollidon^{®} 30; Kollidon^{®} 30 LP; Kollidon^{®} 90 Evo; Kollidon^{®} 90 F; Plasdone^{™} k-25 povidone; Plasdone^{™} k-29 | 32 povidone; Plasdone^{™} k-90 povidone; Plasdone^{™} k-90d povidone; pvp k-30 povidone and pvp k-90.

As used herein, the term "crospovidone" refers to crosslinked polyvinylpyrrolidone, but may also be referred to as crospovidonum, insoluble polyvinylpyrrolidone, or crosslinked PVP. Non-limiting examples of crospovidone may include Kollidon^{®} CL; Kollidon^{®} CL-F; Kollidon^{®} CL-M; and Kollidon^{®} CL-SF.

According to some aspects of the present invention, there is provided a pharmaceutical composition comprising an amorphous solid solution of rivaroxaban and copovidone, povidone or crospovidone, wherein the weight ratio of rivaroxaban to copovidone, povidone or crospovidone may be 1:4.2 to 1:7, 1:4.3 to 1:7, 1:4.4 to 1:7; or 1:4.5 to 1:7.

According to some other aspects of the present invention, there is provided a pharmaceutical composition comprising an amorphous solid solution of rivaroxaban and copovidone, povidone or crospovidone, wherein the weight ratio of rivaroxaban to copovidone, povidone or crospovidone may be 1:4.1, 1:4.2, 1:4.3, 1:4.4, 1:4.5, 1:4.6, 1:4.7, 1:4.8, 1:4.9, 1:5, 1:5.1, 1:5.2, 1:5.3, 1:5.4, 1:5.5, 1:5.6, 1:5.7, 1:5.8, 1:5.9, 1:6, 1:6.1, 1:6.2, 1:6.3, 1:6.4, 1:6.5, 1:6.6, 1:6.7, 1:6.8, 1:6.9, or 1:7. Preferably, there is provided a pharmaceutical composition according to the present invention comprising an amorphous solid solution of rivaroxaban and povidone, wherein the weight ratio of rivaroxaban to povidone may be 1:7.

According to some aspects of the present invention, there is provided a pharmaceutical composition comprising an amorphous solid solution of rivaroxaban and copovidone, povidone or crospovidone, wherein the rivaroxaban may be from 10% to 3%, from 9% to 3%, from 8% to 3%, from 7% to 3%, or from 7% to 4% of the pharmaceutical composition.

According to some other aspects of the present invention, there is provided a pharmaceutical composition comprising an amorphous solid solution of rivaroxaban and copovidone, povidone or crospovidone, wherein the copovidone, povidone or crospovidone may be from 24% to 35%, from 24.5% to 34%, from 25% to 33%, from 26% to 32%, from 27% to 31%, or from 27% to 30% of the pharmaceutical composition.

The present invention also provides a pharmaceutical composition comprising an amorphous solid solution of rivaroxaban and copovidone, wherein the weight ratio of rivaroxaban to copovidone may be 1:4.1 to 1:6.

According to some aspects of the present invention, there is provided a pharmaceutical composition comprising an amorphous solid solution of rivaroxaban and copovidone, wherein the weight ratio of rivaroxaban to copovidone may be 1:4.2 to 1:5.9, 1:4.2 to 1:5.7, 1:4.2 to 1:5.6, 1:4.2 to 1:5.4, 1:4.3 to 1:5.2, 1:4.3 to 1:5.0, 1:4.4 to 1:4.8, 1:4.4 to 1:4.6, or 1:4.4 to 1:4.5.

According to some other aspects of the present invention, there is provided a pharmaceutical composition comprising an amorphous solid solution of rivaroxaban and copovidone, wherein the weight ratio of rivaroxaban to copovidone may be 1:4.1, 1:4.2, 1:4.3, 1:4.4, 1:4.5, 1:4.6, 1:4.7, 1:4.8, 1:4.9, 1:5, 1:5.1, 1:5.2, 1:5.3, 1:5.4, 1:5.5, 1:5.6, 1:5.7, 1:5.8, 1:5.9, or 1:6. Preferably, there is provided a pharmaceutical composition according to the present invention comprising an amorphous solid solution of rivaroxaban and copovidone, wherein the weight ratio of rivaroxaban to copovidone may be 1:4.5.

According to some aspects of the present invention, there is provided a pharmaceutical composition comprising an amorphous solid solution of rivaroxaban and copovidone, wherein the rivaroxaban may be from 10% to 3%, from 7% to 4%, from 7% to 4.5%, from 7% to 5%, from 7% to 6%, or from 6.8% to 6.3% of the pharmaceutical composition.

According to some other aspects of the present invention, there is provided a pharmaceutical composition comprising an amorphous solid solution of rivaroxaban and copovidone, wherein the copovidone may be from 24% to 35%, from 24.5% to 34%, from 25% to 33%, from 26% to 32%, from 27% to 31%, from 28% to 31%, from 29% to 31% or from 29% to 30% of the pharmaceutical composition.

According to an aspect of the present invention, the pharmaceutical composition may further comprise a pharmaceutically acceptable excipient selected from at least one of a filler, disintegrant, glidant and lubricant.

Fillers according to the present invention may be selected from at least one of microcrystalline cellulose, silicified microcrystalline cellulose, lactose, lactose monohydrate, mannitol, cellulose, starch, sorbitol, sucrose, maltodextrin, maltose, magnesium carbonate, magnesium oxide, dibasic anhydrous calcium phosphate, and dibasic dehydrate calcium phosphate. In a preferred embodiment, the filler may be selected from the group consisting of microcrystalline cellulose, silicified microcrystalline cellulose, lactose, lactose monohydrate, or a combination thereof.

According to the present invention, the filler may be from 20% to 70%, from 30% to 65%, from 40% to 60%, or from 50% to 60% of the pharmaceutical composition.

Disintegrants according to the present invention may be selected from at least one of croscarmellose sodium, low-substituted hydroxypropyl cellulose, sodium alginate, calcium carboxymethylcellulose, chitosan hydrochloride, and magnesium aluminium silicate. In a preferred embodiment, the disintegrant may be croscarmellose sodium.

According to the present invention, the disintegrant may be from 0.5% to 10%, from 1% to 9%, from 1% to 8%, from 1.5% to 7%, from 1.5% to 6%, from 2% to 5%, from 2% to 4%, or from 2% to 3% of the pharmaceutical composition.

Glidants according to the present invention may be selected from at least one of colloidal silicon dioxide, amorphous anhydrous colloidal silicon dioxide, talc, tribasic calcium silicate, calcium silicate, silica, magnesium trisilicate, silicified cellulose, hydrophobic colloidal silica and Aerosil^{®} 200. In a preferred embodiment of the present invention, the glidant may be colloidal silicon dioxide, and preferably amorphous anhydrous colloidal silicon dioxide.

According to the present invention, the glidant may be from 0.5% to 10%, from 0.5% to 8%, from 0.5% to 6%, from 1% to 5%, from 1% to 4%, from 1% to 3%, or from 1% to 2% of the pharmaceutical composition.

Lubricants according to the present invention may be selected from at least one of sodium stearyl fumarate, sodium lauryl sulphate, magnesium lauryl sulphate, stearic acid, calcium stearate, glyceryl dibehenate, glyceryl monostearate, glyceryl tristearate, palmitic acid, sorbitan monolaurate, sorbitan monopalmitate, zinc stearate, sorbitan sesquioleate, magnesium stearate and sorbitan trioleate. In an embodiment according to the present invention, the lubricant may be selected from the group consisting of sodium stearyl fumarate, sodium lauryl sulphate or a combination thereof.

According to the present invention, the lubricant may be from 0.1% to 10%, from 0.5% to 10%, from 0.75% to 9%, from 1% to 8%, from 1.25% to 7, from 1.50% to 6%, or from 1.75% to 5% of the pharmaceutical composition.

According to the present invention, the pharmaceutical composition may comprise a therapeutically effective amount of rivaroxaban, that may result in at least prevention or delay of onset or amelioration or attainment of symptoms.

According to some embodiments of the present invention, the pharmaceutical composition may comprise rivaroxaban in an amount between 2.5 mg to 30 mg, 5 mg to 27.5 mg, 7.5 mg to 25 mg, 10 mg to 22.5 mg, 12.5 mg to 20 mg, or 15 mg to 17.5 mg.

According to some specific embodiments of the present invention, the pharmaceutical composition may comprise rivaroxaban in an amount of 2.5 mg, 10 mg, 15 mg, 20 mg, or 30 mg.

In an embodiment of the present invention, the pharmaceutical composition may comprise:
a) an amorphous solid solution of rivaroxaban and povidone in a weight ratio of 1:6 to 1:7;
b) from 60% to 70% of filler;
c) from 3% to 8% of disintegrant; and
d) from 0.1% to 1% of lubricant.

In a preferred embodiment of the present invention, the pharmaceutical composition may comprise:
a) an amorphous solid solution of rivaroxaban and copovidone in a weight ratio of 1:4.1 to 1:6;
b) from 20% to 70% of filler;
c) from 0.5% to 10% of disintegrant;
d) from 0.5% to 10% of glidant;
e) from 0.5% to 10%, of lubricant.

In a further preferred embodiment of the present invention, the pharmaceutical composition may comprise:
a) an amorphous solid solution of rivaroxaban and copovidone in a weight ratio of 1:4.2 to 1:5.6;
b) from 30% to 65% of filler;
c) from 1% to 8% of disintegrant;
d) from 1% to 5% of glidant;
e) from 1% to 8%, of lubricant.

In yet a further preferred embodiment of the present invention, the pharmaceutical composition may comprise:
a) an amorphous solid solution of rivaroxaban and copovidone in a weight ratio of 1:4.4 to 1:4.6;
b) from 50% to 60% of filler;
c) from 2% to 4% of disintegrant;
d) from 1% to 3% of glidant;
e) from 1.5% to 6%, of lubricant.

Unless otherwise specified, the % of the components of the pharmaceutical composition refer to percentages by weight based on the total weight of the composition.

Moreover, unless specifically stated otherwise, where the pharmaceutical composition is coated or filled into a capsule, it is to be understood that the reference to % of the components refers to the weight percentage based on the total weight of the pharmaceutical composition i.e. excluding the weight percentages of the coating or capsule.

In an aspect of the invention, there is provided a pharmaceutical composition comprising an amorphous solid solution of rivaroxaban and copovidone, povidone or crospovidone for use in the treatment of a thromboembolic event.

In a further aspect of the invention, there is provided a pharmaceutical composition comprising an amorphous solid solution of rivaroxaban and copovidone for use in the treatment of a thromboembolic event.

In yet a further aspect of the present invention, there is provided a method for treating or preventing a thromboembolic event in a subject, the method comprising administering to a subject in need thereof a therapeutically effective amount of a composition comprising an amorphous solid solution of rivaroxaban and copovidone, povidone or crospovidone.

In an aspect of the present invention, there is provided a method for treating or preventing a thromboembolic event in a subject, the method comprising administering to a subject in need thereof a therapeutically effective amount of a composition comprising an amorphous solid solution of rivaroxaban and copovidone.

The present invention also relates to the use of a composition comprising an amorphous solid solution of rivaroxaban and copovidone, povidone or crospovidone, for the manufacture of a medicament for the therapeutic and/or prophylactic treatment of a thromboembolic event.

The present invention further relates to the use of a composition comprising an amorphous solid solution of rivaroxaban and copovidone, for the manufacture of a medicament for the therapeutic and/or prophylactic treatment of a thromboembolic event.

As used herein, the term "treatment" includes the therapeutic and/or prophylactic treatment of a thromboembolic event.

As used herein, the term "thromboembolic event" includes acute coronary syndrome spectrum as ST Segment Elevation Myocardial Infarction (STEMI) (also known as Q-wave MI), Non ST Segment Elevation Myocardial Infarction (NSTEMI) (also known as Non Q-wave MI) and unstable angina (UA), as well as stable angina pectoris, vascular re-occlusions and restenosis after angioplasty or aorto-coronary bypass, peripheral arterial occlusion disorders, pulmonary embolism, or deep vein thrombosis, renal thrombosis, transitory ischaemic attacks and stroke, inhibition of tumor growth and development of metastasis, treatment of disseminated intravascular coagulation (DIC) and the so called "economy class syndrome", especially in patients with risk of venous thrombosis, atherosclerotic diseases, inflammatory diseases, as rheumatic diseases of the musculoskeletal system, Alzheimer's disease, inhibition of old-age macula-degeneration, diabetic retinopathy, diabetic nephropathy and other microvascular diseases.

Included are also events derived from cardiogenic thromboembolism, for instance cerebral ischemic diseases, stroke, systemic embolism and ischemic attacks, especially in patients with acute, intermittent or persistent arrhythmia of the heart such as atrial fibrillation or alongside cardioversion, or in patients with valvular heart disease or artificial heart valves.

Included are also events derived from thromboembolic complications which can arise within patients with microangiopathic hemolytic anaemia, extracorporal circulation such as hemodialysis, or prosthetic heart valves as well as from thromboembolic complication, e.g. venous thromboembolism in tumor patients, in particular in patients undergoing surgical interventions, chemotherapy or radiotherapy.

In an aspect of the invention, there is provided a pharmaceutical composition comprising an amorphous solid solution of rivaroxaban and copovidone, povidone or crospovidone for use in the treatment of a subject suffering from at least one of deep vein thrombosis and pulmonary embolism, wherein the subject may be in a fasted state. The pharmaceutical composition of the present invention and the pharmaceutical composition for use according to the present invention may be administered orally.

In a specific aspect of the invention, there is provided a pharmaceutical composition comprising an amorphous solid solution of rivaroxaban and copovidone for use in the treatment of a subject suffering from at least one of deep vein thrombosis and pulmonary embolism, wherein the subject may be in a fasted state. The pharmaceutical composition of the present invention and the pharmaceutical composition for use according to the present invention may be administered orally.

Surprisingly, it has been found that the pharmaceutical composition of the present invention has a significantly improved dissolution of rivaroxaban. In particular, it was shown that the pharmaceutical composition of the invention did not require the presence of food to enhance the dissolution of rivaroxaban. The dissolution rate of rivaroxaban from the pharmaceutical composition of the present invention was shown, in subjects in a fasted state, to be surprisingly comparable to that of the reference product (Xarelto^{®}) in subjects in a fed state.

Advantageously, it was found that the pharmaceutical composition of the present invention showed bioequivalence in a fasted state to the originator drug product Xarelto^{®} in a fed state. As used herein, the term "bioequivalence" refers to two preparations of the same active ingredient showing substantially the same pharmacokinetic profile. The "fasted state" as used herein refers to a state where the subject is provided with no food for at least 10 hours, preferably 10 to 15 hours, more preferably 10 to 12 hours prior to administration of the drug. The "fed state" as used herein refers to a subject who has had a meal within the proceeding 4 hours, preferably 3 hours, preferably 2 hours, preferably 1 hour, preferably 45 mins, preferably 30 mins prior to administration of the drug.

In bioequivalence studies, a drug kinetic profile can be summarized by the pharmacokinetics parameters Cₘₐₓ and area under the curve (AUC).

Cₘₐₓ is the maximum or peak plasma concentration that a drug achieves in a specified test area of the body after the drug has been administered.

The AUC is the plasma concentration that a drug achieves in a specified test area of the body after the drug has been administered over a period of time.

The AUC is dependent on the amount of drug that enters into systemic circulation and the ability of that system to eliminate the drug. Standard calculation of AUC considers the AUC from time 0 which corresponds to the time when the drug is administered to "t", which represents the time of the last measurable concentration of the drug. This is called AUC₀₋ₜ and represents the observed exposure to a drug. The total AUC or AUC_{o-∞} is the area under the curve from time 0 extrapolated to an infinite time.

Cₘₐₓ and AUC are the most reliable parameters to assess the bioavailability of a drug and/or the bioequivalence of two compositions comprising the same drug.

The pharmaceutical composition of the present invention and for use according to the present invention may achieve an AUC₀₋ₜ in the subject from 1,600 to 3,500 ng*h/ml, 1,700 to 3,400 ng*h/ml, 1,800 to 3,300 ng*h/ml, 1,900 to 3,300 ng*h/ml 2,000 to 3,200 ng*h/ml, 2,100 to 3,100 ng*h/ml, 2,200 to 3,000 ng*h/ml, 2,300 to 2,900 ng*h/ml, or 2,400 to 2,700 ng*h/ml 48 hours after administration.

The pharmaceutical composition of the present invention and for use according to the present invention may achieve a Cₘₐₓ in the subject from 200 to 550 ng/ml, 200 to 500 ng/ml, 250 to 450 ng/ml, 275 to 400 ng/ml or 300 to 400 ng/ml.

The pharmaceutical composition of the present invention and for use according to the present invention may be bioequivalent to Xarelto^{®} when Xarelto^{®} is administered to a subject in a fed state.

In an embodiment, the pharmaceutical composition according to the present invention may be formulated as a single dosage form.

The pharmaceutical composition according to the present invention may be formulated for oral administration. The composition may be formulated as a tablet such as mono-layered tablets, bilayered tablets, trilayered tablet, multilayer tablet, caplets, minitablets, microtablets, capsules, tablet in tablet, tablets in a capsule, microtablets in a capsule, minitablets in a capsule, granules in a capsule, pellets, pellets in a capsule, powder, granules, extrudes, pellets, beads or spheroids, suspension or any other suitable dosage form meant for oral administration to a patient. In a preferred embodiment, the pharmaceutical composition may be formulated as a capsule, and more preferably, the capsule may be a hard hypromellose capsule.

Advantageously, it was found that the pharmaceutical composition of the present invention and Xarelto^{®} are bioequivalent, as shown by the fact that both AUC and Cₘₐₓ fell within the range 80% - 125% based on the 90% confidence interval of the ratio of a In-transformed exposure measurement.

Amorphous solid solutions can be manufactured by several methods, involving breaking the lattice structure of the crystalline drug, which is converted into a liquid state by applying heat or dissolving it in a solvent. The system is then rapidly cooled or dried to generate a solid drug in an amorphous state which is then stabilised with a polymer.

The hot melt extrusion process for preparing an amorphous solid solution requires that the drug and the polymer are mixed, melted, dispersed and then extruded.

In an embodiment, a hot melt extrusion method for preparing the pharmaceutical composition according to the present invention may comprise the steps of:
a) melting rivaroxaban and copovidone, povidone or crospovidone at a process temperature of 150 °C to 220 °C to form a hot melt mixture;
b) homogenising the hot melt mixture; and
c) combining the hot melt mixture with at least one further pharmaceutically acceptable excipient.

In an embodiment, a hot melt extrusion method for preparing the pharmaceutical composition according to the present invention may comprise the steps of:
a) melting rivaroxaban and povidone at a process temperature of 150 °C to 220 °C to form a hot melt mixture;
b) homogenising the hot melt mixture; and
c) combining the hot melt mixture with at least one further pharmaceutically acceptable excipient.

In an embodiment, a hot melt extrusion method for preparing the pharmaceutical composition according to the present invention may comprise the steps of:
a) melting rivaroxaban and copovidone at a process temperature of 150 °C to 220 °C to form a hot melt mixture;
b) homogenising the hot melt mixture; and
c) combining the hot melt mixture with at least one further pharmaceutically acceptable excipient.

The present invention is now described in more detail by, but not limited to, the following Examples.

### Example 1 - Formulations of prototypes 3A, 3B, 3C

### Prototype 3A

| **Components** | **Amount (mg)** | **Percentage in weight (%)** |
|---|---|---|
| **Rivaroxaban HME (hot melt extrusion) ingredients** | | |
| **Rivaroxaban** | 20.0 | 6.7 |
| **Copovidone Kollidon VA64** | 90.0 | 30.0 |

| **Intragranular ingredients** | | |
|---|---|---|
| **Lactose, monohydrate** | 72.0 | 24.0 |
| **Microcrystalline cellulose, Silicified** | 100.0 | 33.3 |
| **Sodium stearyl fumarate** | 3.0 | 1.0 |

| **Extragranular ingredients** | | |
|---|---|---|
| **Croscarmellose sodium** | 8.0 | 2.7 |
| **Aerosil 200** | 4.0 | 1.3 |
| **Sodium stearyl fumarate** | 3.0 | 1.0 |
| **Total of encapsulating material** | 300.0 | 100 |

| **Capsules** | | |
|---|---|---|
| **Hard hypromellose capsules** | - | - |

### Prototype 3B

| **Components** | **Amount (mg)** | **Percentage in weight (%)** |
|---|---|---|
| **Rivaroxaban HME (hot melt extrusion) ingredients** | | |
| **Rivaroxaban** | 20.0 | 6.7 |
| **Copovidone Kollidon VA64** | 90.0 | 30.0 |

| **Intragranular ingredients** | | |
|---|---|---|
| **Microcrystalline cellulose, Silicified** | 50 | 16.7 |
| **Sodium stearyl fumarate** | 3.0 | 1.0 |

| **Extragranular ingredients** | | |
|---|---|---|
| **Lactose, monohydrate** | 72.0 | 24.0 |
| **Microcrystalline cellulose, Silicified** | 50.0 | 16.7 |
| **Croscarmellose sodium** | 8.0 | 2.7 |
| **Aerosil 200** | 4.0 | 1.3 |
| **Sodium stearyl fumarate** | 3.0 | 1.0 |
| **Total of encapsulating material** | 300.0 | 100 |

| **Capsules** | | |
|---|---|---|
| **Hard hypromellose capsules** | - | - |

### Prototype 3C

| **Components** | **Amount (mg)** | **Percentage in weight (%)** |
|---|---|---|
| **Rivaroxaban HME (hot melt extrusion) ingredients** | | |
| **Rivaroxaban** | 20.0 | 6.5 |
| **Copovidone Kollidon VA64** | 90.0 | 29.3 |

| **Intragranular ingredients** | | |
|---|---|---|
| **Lactose, monohydrate** | 72.0 | 23.4 |
| **Microcrystalline cellulose, Silicified** | 100.0 | 32.5 |
| **Croscarmellose sodium** | 4.0 | 1.3 |
| **Sodium lauryl sulphate** | 7.5 | 2.4 |
| **Sodium stearyl fumarate** | 3.0 | 1.0 |

| **Extragranular ingredients** | | |
|---|---|---|
| **Croscarmellose sodium** | 4.0 | 1.3 |
| **Aerosil 200** | 4.0 | 1.3 |
| **Sodium stearyl fumarate** | 3.0 | 1.0 |
| **Total of encapsulating material** | 307.5 | 100 |

| **Capsules** | | |
|---|---|---|
| **Hard hypromellose capsules** | - | - |

The rivaroxaban HME ingredients, intragranular ingredients and extragranular ingredients are filled into hard hypromellose capsules.

### Example 2 - In vivo tests conducted on humans

The three formulations (*i.e*., Prototypes 3A, 3B and 3C) were each tested in a human bioavailability study, performed as a randomized, single dose, cross-over, open-label study on twenty healthy human volunteers. The objective of this study was to evaluate bioequivalence of the three formulations containing rivaroxaban (*i.e*., Prototypes 3A, 3B and 3C) and compare them to the reference Xarelto^{®}.

Each subject received a single oral dose of:
- Rivaroxaban 20 mg (Prototype 3A) in fasted state,
- Rivaroxaban 20 mg (Prototype 3B) in fasted state,
- Rivaroxaban 20 mg (Prototype 3C) in fasted state, or
- Rivaroxaban 20 mg (Xarelto^{®}) in fed state.

The fasted state consisted of 10 to 12 hours of overnight fasting before the administration of the dose on the second day of each test period.

The fed state consisted of the consumption of a standardized high-fat high-calorie breakfast in the morning of the second day of each test period 30 minutes before administration of the dose.

Pharmacokinetic parameters - area under the concentration-time curve from zero to "t", where "t" represents the time of the last measurable concentration of the drug (AUC₀₋ₜ), area under the concentration-time curve from zero to infinity (AUC_{0-∞}) and maximum serum concentration (Cₘₐₓ) were evaluated. The natural logarithmic transformation of AUC₀₋ₜ, AUC_{0-∞} and Cₘₐₓ was used for all statistical inference. AUC₀₋ₜ was calculated using the trapezoidal rule, while Cₘₐₓ was taken directly from the observed data. AUC_{0-∞} was calculated from the sum of AUC₀₋ₜ and extrapolated AUC from last measured concentration to infinity, calculated as Clast/terminal elimination rate constant, where terminal elimination rate constant was estimated for each subject and for each treatment via linear regression of at least three last points at the terminal phase of the log-concentration versus time curve. The pharmacokinetic parameters were analyzed using an ANOVA (analysis of variance) model where the effects of subject, treatment, period, and sequence were included as the fixed factors. The 90% confidence interval for the ratio of least-squares means between each test and reference products was calculated. Descriptive statistics, included arithmetic mean, median, maximum and minimum values and standard deviation (SD).

The pharmacokinetic parameters Cₘₐₓ, AUC₀₋ₜ and AUC_{0-∞} were evaluated and are listed in **Table 1** below:

**Table 1: Results of pharmacokinetic parameters Cₘₐₓ, AUC₀₋ₜ and AUC_{0-∞} of Prototypes 3A, 3B, 3C and reference (Xarelto^{®})**

| **Pharmacokinetic parameter** | **Prototype 3A (Mean ± SD)** | **Prototype 3B (Mean ± SD)** | **Prototype 3C (Mean ± SD)** | **Reference (Xarelto^{®}) (Mean ± SD)** |
|---|---|---|---|---|
| **Cₘₐₓ (ng/ml)** | 352.276 ± 103.530 | 356.986 ± 120.909 | 373.251 ± 126.505 | 334.884 ± 49.046 |
| **AUC₀₋ₜ (ng*h/ml)** | 2525.23 ± 734.33 | 2592.76 ± 757.23 | 2571.44 ± 751.83 | 2591.83 ± 575.92 |
| **AUC_{0-∞} (ng*h/ml)** | 2576.39 ± 754.51 | 2662.94 ± 796.36 | 2634.38 ± 755.47 | 2622.02 ± 589.32 |

The point estimate and the 90% confidence intervals of the ratios for the In-transformed Cₘₐₓ, AUC₀₋ₜ and AUC_{0-∞} of rivaroxaban are listed in **Tables 2, 3** and **4.**

**Table 2: Point estimate and 90% confidence interval for rivaroxaban in Prototype 3A vs Xarelto^{®}**

| **Pharmacokinetic parameter** | **Point Estimate** | **Lower Limit** | **Upper Limit** |
|---|---|---|---|
| **Cₘₐₓ (ng/ml)** | 103.62 | 92.55 | 116.01 |
| **AUC₀₋ₜ (ng*h/ml)** | 95.93 | 88.36 | 104.16 |
| **AUC_{0-∞} (ng*h/ml)** | 96.80 | 89.24 | 104.99 |

**Table 3: Point estimate and 90% confidence interval for rivaroxaban in Prototype 3B vs Xarelto^{®}**

| **Pharmacokinetic parameter** | **Point Estimate** | **Lower Limit** | **Upper Limit** |
|---|---|---|---|
| **Cₘₐₓ (ng/ml)** | 103.64 | 90.89 | 118.17 |
| **AUC₀₋ₜ (ng*h/ml)** | 98.96 | 90.82 | 107.83 |
| **AUC_{0-∞} (ng*h/ml)** | 100.41 | 92.07 | 109.49 |

**Table 4: Point estimate and 90% confidence interval for rivaroxaban in Prototype 3C vs Xarelto^{®}**

| **Pharmacokinetic parameter** | **Point Estimate** | **Lower Limit** | **Upper Limit** |
|---|---|---|---|
| **Cₘₐₓ (ng/ml)** | 106.95 | 92.04 | 124.27 |
| **AUC₀₋ₜ (ng*h/ml)** | 97.03 | 87.19 | 107.98 |
| **AUC_{0-∞} (ng.h/ml)** | 98.29 | 88.41 | 109.27 |

All three prototypes displayed Cₘₐₓ and AUC parameters within the standard bioequivalence range of 80% - 125%.

### Example 3 - Dissolution study of Prototypes 3A and 3B vs Xarelto^{®}.

A study was conducted to compare the dissolution profiles of prototypes 3A and 3B against the reference listed drug (Xarelto^{®} 20 mg and 10 mg).

### Preparation of FeSSIF dissolution media, pH 5.0

Fed conditions were simulated using dissolution media FeSSIF (fed state simulated intestinal fluid (human)). FeSSIF dissolution media, pH 5.0 is prepared according to the following method using SIF (simulated intestinal fluid) powder commercially available from Biorelevant:
Step 1: preparation of acetate buffer for FeSSIF (1 Litre):
   Dissolve 4.04 g of NaOH (pellets), 8.65 g of Glacial Acetic Acid, 11.87 g of NaCl in 0.9 L of purified water; and
   Adjust the pH to 5.0 with either 1 N NaOH or 1 N HCl and make up to volume (1 L) with purified water.
Step 2: to make 1 litre of FeSSIF:
   Add 11.2 g of SIF powder to approximately 500 mL of buffer;
   Stir until SIF powder has dissolved; and
   Make up to volume (1 L) with the buffer.

### Preparation of FaSSIF dissolution media, pH 6.5

Fasted conditions were simulated using dissolution media FaSSIF (fasted state simulated intestinal fluid (human)). FaSSIF dissolution media, pH 6.5 is prepared according to the following method using SIF (simulated intestinal fluid) powder commercially available from Biorelevant:
Step 1: preparation of phosphate buffer for FaSSIF (1 Litre):
   Dissolve 0.42 g NaOH (pellets), 3.95 g of NaH₂PO₄*H₂O (monohydrate), 6.19 g of NaCl in 0.9 L of purified water; and
   Adjust the pH to 6.5 with either 1 N NaOH or 1 N HCl and make up to volume (1 L) with purified water
Step 2: to make 1 litre of FaSSIF:
   Add 2.24 g of SIF Powder to approximately 500 mL of buffer;
   Stir until SIF Powder has dissolved; and
   Make up to volume (1 L) with the buffer.

Equilibrate the solution for 2 hours until it becomes slightly opalescent.

### Dissolution Experiment

The study was conducted using standard USP 2 dissolution bath equipped with a paddle apparatus using equipment from Sotax (Aesch, Switzerland). The dissolution medium (either 900 mL of FaSSIF buffer, pH 6.5 or 900 mL of FeSSIF buffer, pH 5.0 prepared in accordance with the method described above) was preheated to 37°C and the stirrer speed was set to 75 RPM. After 45 min, the agitation speed was raised to 150 RPM and stirred for a further 15 mins.

Formulations (Xarelto^{®} 20 mg or Xarelto^{®} 10 mg) and the capsule content of formulations (prototype 3A, prototype 3B) were administered directly into dissolution vessel. The amount of dissolved active pharmaceutical ingredient in dissolution medium was monitored by UV spectroscopy at 280 nm.

### Dissolution Results

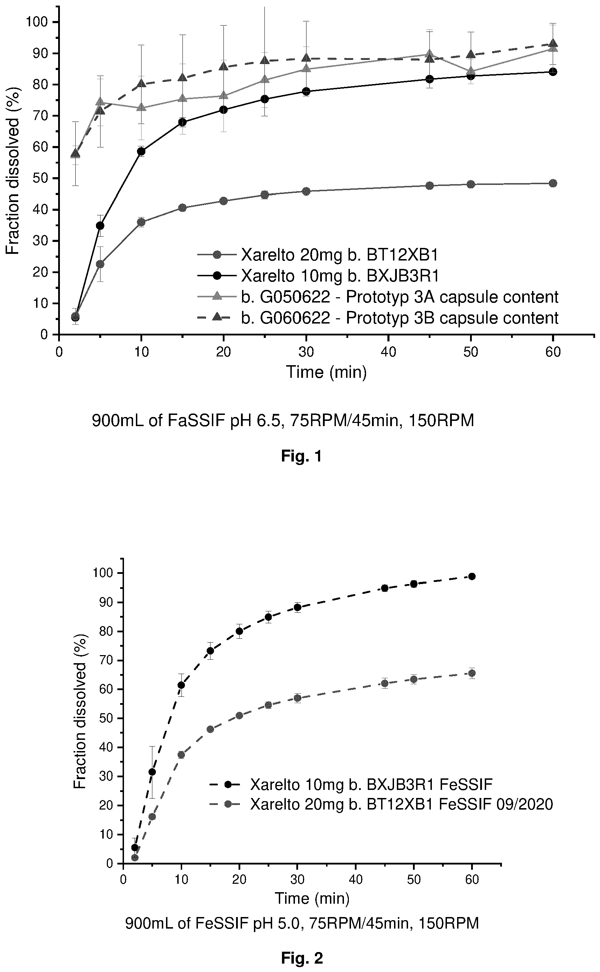

Comparison of Fig. 1 and Fig. 2 shows that Xarelto^{®} 20 mg and Xarelto^{®} 10 mg demonstrate a higher dissolution profile in the fed state than the fasted state.

Fig.2 demonstrates that prototypes 3A and 3B exhibit dissolution profiles matching Xarelto^{®} 10 mg even under fasted simulated conditions. This indicates that there is no food effect.

### Example 4 - Formulation of prototype 4A

### Prototype 4A

| **Components** | **Amount (mg)** | **Percentage in weight (%)** |
|---|---|---|
| **Rivaroxaban HME (hot melt extrusion) ingredients** | | |
| **Rivaroxaban** | 20.0 | 4.0 |
| **Povidone (Kollidon 17 PF)** | 140.0 | 28.0 |

| **Additional excipients** | | |
|---|---|---|
| **Microcrystalline cellulose** | 312.5 | 62.5 |
| **Croscarmellose sodium** | 25.0 | 5.0 |
| **Magnesium stearate** | 2.5 | 0.5 |

An amorphous solid dispersion (ASD) comprising rivaroxaban and Povidone (Kollidon 17 PF) was prepared by a hot melt extrusion (HME) process. Rivaroxaban was prepared by HME at 190 °C and Povidone (Kollidon 17 PF) was prepared by HME at 175 °C. The rivaroxaban and Povidone (Kollidon 17 PF) were then milled on a ball mill and sieved through a 200 µm sieve.

The formulation of Prototype 4A was then prepared by homogenization of the ASD obtained above with microcrystalline cellulose and croscarmellose for 10 min in turbula, then adding magnesium stearate and homogenizing for 5 min. The formulation was then formed into a tablet by direct tabletting at 3 kN.

### Dissolution Results

A dissolution experiment with the formulation of Prototype 4A was carried out.

The dissolution results in phosphate buffer pH 6.8 without surfactant addition - Sotax USP II, 900 ml are provided below:

| **Time (mins)** | **Concentration (µg/ml)** | **% of API release** |
|---|---|---|
| 0 | 0 | 0 |
| 5 | 13.00002 | 58.55863 |
| 10 | 14.23103 | 64.10373 |
| 20 | 14.79864 | 66.66056 |
| 30 | 14.93344 | 67.26776 |
| 40 | 15.0227 | 67.6698 |
| 50 | 15.07881 | 67.92258 |
| 60 | 15.12006 | 68.10837 |
| 70 | 15.16436 | 68.30794 |
| 80 | 15.18314 | 68.39252 |
| 90 | 15.18219 | 68.38824 |

Below, there is provided a non-exhaustive list of non-limiting embodiments. Any one or more of the features of these embodiments may be combined with any one or more features of another embodiment or aspect described herein.

Embodiment 1: a pharmaceutical composition comprising an amorphous solid solution of rivaroxaban and copovidone, wherein the weight ratio of rivaroxaban to copovidone is 1:4.1 to 1:6.

Embodiment 2: the pharmaceutical composition according to Embodiment 1, further comprising a pharmaceutically acceptable excipient selected from at least one of filler, disintegrant, glidant and lubricant.

Embodiment 3: the pharmaceutical composition according to Embodiment 2, wherein the filler is selected from at least one of microcrystalline cellulose, silicified microcrystalline cellulose, lactose, lactose monohydrate, mannitol, cellulose, starch, sorbitol, sucrose, maltodextrin, maltose, magnesium carbonate, magnesium oxide, dibasic anhydrous calcium phosphate, and dibasic dehydrate calcium phosphate.

Embodiment 4: the pharmaceutical composition according to Embodiment 2 or 3, wherein the filler is from 20% to 70%, from 30% to 65%, from 40% to 60%, or from 50% to 60% of the pharmaceutical composition.

Embodiment 5: the pharmaceutical composition according to Embodiment 2, wherein the disintegrant is selected from at least one of croscarmellose sodium, low-substituted hydroxypropyl cellulose, sodium alginate, calcium carboxymethylcellulose, chitosan hydrochloride, and magnesium aluminium silicate.

Embodiment 6: the pharmaceutical composition according to Embodiment 2 or 5, wherein the disintegrant is from 0.5% to 10%, from 1% to 9%, from 1% to 8%, from 1.5% to 7%, from 1.5% to 6%, from 2% to 5%, from 2% to 4%, or from 2% to 3% of the pharmaceutical composition..

Embodiment 7: the pharmaceutical composition according to Embodiment 2, wherein the glidant is selected from at least one of colloidal silicon dioxide, amorphous anhydrous colloidal silicon dioxide, talc, tribasic calcium silicate, calcium silicate, silica, magnesium trisilicate, silicified cellulose and hydrophobic colloidal silica.

Embodiment 8: the pharmaceutical composition according to Embodiment 2 or 7, wherein the glidant is from 0.5% to 10%, from 0.5% to 8%, from 0.5% to 6%, from 1% to 5%, from 1% to 4%, from 1% to 3%, or from 1% to 2% of the pharmaceutical composition.

Embodiment 9: the pharmaceutical composition according to Embodiment 2, wherein the lubricant is selected from at least one of sodium stearyl fumarate, sodium lauryl sulphate, magnesium lauryl sulphate, stearic acid, calcium stearate, glyceryl dibehenate, glyceryl monostearate, glyceryl tristearate, palmitic acid, sorbitan monolaurate, sorbitan monopalmitate, zinc stearate, sorbitan sesquioleate, magnesium stearate and sorbitan trioleate.

Embodiment 10: the pharmaceutical composition according to Embodiment 2 or 9, wherein the lubricant is from 0.5% to 10%, from 0.75% to 9%, from 1% to 8%, from 1.25% to 7, from 1.50% to 6%, or from 1.75% to 5% of the pharmaceutical composition.

Embodiment 11: the pharmaceutical composition according to any preceding Embodiment, wherein the rivaroxaban is in amount of 2.5 mg, 10 mg, 15 mg, 20 mg, or 30 mg.

Embodiment 12: a pharmaceutical composition comprising:
an amorphous solid solution of rivaroxaban and copovidone in a weight ratio of 1:4.1 to 1:6;
from 20% to 70% of filler;
from 0.5% to 10% of disintegrant;
from 0.5% to 10% of glidant;
from 0.5% to 10%, of lubricant.

Embodiment 13: the pharmaceutical composition according to any preceding Embodiment for use in the treatment of a subject suffering from at least one of deep vein thrombosis and pulmonary embolism.

Embodiment 14: the pharmaceutical composition for use according to Embodiment 13, wherein the subject is in a fasted state.

Embodiment 15: the pharmaceutically composition according to Embodiments 1 to 12 and the pharmaceutical composition for use according to Embodiment 13 or 14, wherein the pharmaceutical composition is administered orally.

Embodiment 16: the pharmaceutical composition according to Embodiments 1 to 12 and the pharmaceutical composition for use according to Embodiments 13 to 15, wherein the pharmaceutical composition achieves an AUC₀₋ₜ in the subject from 1,600 to 3,500 ng*h/ml 48 hours after administration.

Embodiment 17: the pharmaceutical composition according to Embodiments 1 to 12 and the pharmaceutical composition for use according to Embodiments 13 to 16, wherein the pharmaceutical composition achieves a Cₘₐₓ in the subject from 200 to 550 ng/ml.

Embodiment 18: the pharmaceutical composition according to Embodiments 1 to 12 and the pharmaceutical composition for use according to Embodiments 13 to 17, wherein the pharmaceutical composition is bioequivalent to Xarelto^{®} when Xarelto^{®} is administered to a subject in a fed state.

Embodiment 19: a hot melt extrusion method for preparing the pharmaceutical composition according to any one of Embodiments 1 to 18 comprising the steps of:
a) melting rivaroxaban and copovidone at a process temperature of 150 °C to 220 °C to form a hot melt mixture;
b) homogenising the hot melt mixture; and
c) combining the hot melt mixture with at least one further pharmaceutically acceptable excipient.

## Claims

1. A pharmaceutical composition comprising an amorphous solid solution of rivaroxaban and copovidone, povidone or crospovidone wherein the weight ratio of rivaroxaban to copovidone, povidone or crospovidone is 1:4.1 to 1:7.

2. The pharmaceutical composition according to claim 1, further comprising a pharmaceutically acceptable excipient selected from at least one of filler, disintegrant, glidant and lubricant.

3. The pharmaceutical composition according to claim 2, wherein the filler is selected from at least one of microcrystalline cellulose, silicified microcrystalline cellulose, lactose, lactose monohydrate, mannitol, cellulose, starch, sorbitol, sucrose, maltodextrin, maltose, magnesium carbonate, magnesium oxide, dibasic anhydrous calcium phosphate, and dibasic dehydrate calcium phosphate.

4. The pharmaceutical composition according to claim 2 or 3, wherein the filler is from 20% to 70%, or from 30% to 65% of the pharmaceutical composition.

5. The pharmaceutical composition according to claim 2, wherein the disintegrant is selected from at least one of croscarmellose sodium, low-substituted hydroxypropyl cellulose, sodium alginate, calcium carboxymethylcellulose, chitosan hydrochloride, and magnesium aluminium silicate.

6. The pharmaceutical composition according to claim 2 or 5, wherein the disintegrant is from 0.5% to 10%, from 1% to 9%, from 1% to 8%, from 1.5% to 7%, from 1.5% to 6%, or from 2% to 5% of the pharmaceutical composition..

7. The pharmaceutical composition according to claim 2, wherein the glidant is selected from at least one of colloidal silicon dioxide, amorphous anhydrous colloidal silicon dioxide, talc, tribasic calcium silicate, calcium silicate, silica, magnesium trisilicate, silicified cellulose and hydrophobic colloidal silica.

8. The pharmaceutical composition according to claim 2 or 7, wherein the glidant is from 0.5% to 10%, from 0.5% to 8%, from 0.5% to 6%, from 1% to 5%, from 1% to 4%, from 1% to 3%, or from 1% to 2% of the pharmaceutical composition.

9. The pharmaceutical composition according to claim 2, wherein the lubricant is selected from at least one of sodium stearyl fumarate, sodium lauryl sulphate, magnesium lauryl sulphate, stearic acid, calcium stearate, glyceryl dibehenate, glyceryl monostearate, glyceryl tristearate, palmitic acid, sorbitan monolaurate, sorbitan monopalmitate, zinc stearate, sorbitan sesquioleate, magnesium stearate and sorbitan trioleate.

10. The pharmaceutical composition according to claim 2 or 9, wherein the lubricant is from 0.5% to 10% of the pharmaceutical composition.

11. The pharmaceutical composition according to any one of the preceding claims, wherein the rivaroxaban is in an amount of 2.5 mg, 10 mg, 15 mg, 20 mg, or 30 mg.

12. The pharmaceutical composition according to any one of the preceding claims for use in the treatment of a subject suffering from at least one of deep vein thrombosis and pulmonary embolism, optionally wherein the subject is in a fasted state, and optionally wherein the pharmaceutical composition is administered orally.

13. The pharmaceutical composition according to any one of claims 1 to 11 or the pharmaceutical composition for use according to claim 12, wherein the pharmaceutical composition achieves an AUC₀₋ₜ in the subject from 1,600 to 3,500 ng*h/ml 48 hours after administration.

14. The pharmaceutical composition according to any one of claims 1 to 11 or the pharmaceutical composition for use according to claim 12 or 13, wherein the pharmaceutical composition achieves a Cₘₐₓ in the subject from 200 to 550 ng/ml, or wherein the pharmaceutical composition is bioequivalent to Xarelto^{®} when Xarelto^{®} is administered to a subject in a fed state.

15. A hot melt extrusion method for preparing the pharmaceutical composition according to anyone of claims 1 to 11 comprising the steps of:
a) melting rivaroxaban and copovidone, povidone or crospovidone at a process temperature of 150 °C to 220 °C to form a hot melt mixture;
b) homogenising the hot melt mixture; and
c) combining the hot melt mixture with at least one further pharmaceutically acceptable excipient.
